(19) Europäisches Patentamt
European Patent Office
Office européen des brevets

(11) Publication number: **0 276 154 A2**

# (12) EUROPEAN PATENT APPLICATION

(21) Application number: **88300484.8**

(51) Int. Cl.[4]: **C 12 M 3/00**

(22) Date of filing: **21.01.88**

(30) Priority: **23.01.87 US 7072**

(43) Date of publication of application:
**27.07.88 Bulletin 88/30**

(84) Designated Contracting States:
**CH DE FR GB LI SE**

(71) Applicant: **BELLCO GLASS, INC.**
**340 Edrudo Road**
**Vineland New Jersey 08360 (US)**

(72) Inventor: **Familletti, Phillip C., Dr.**
**24 Clover Hill Road**
**Millington, New Jersey 07946 (US)**

(74) Representative: **Oliver, Roy Edward et al**
**POLLAK MERCER & TENCH High Holborn House 52-54**
**High Holborn**
**London WC1V 6RY (GB)**

(54) **Method and apparatus for cell culture.**

(57) Methods and apparatus are provided for growing both suspension type cells and anchorage-dependent cells in airlift bioreactors. A packing means, such as stainless steel wool, is provided in the growth chamber of the bioreactor to increase cell density by trapping suspension cells or acting as a solid support for anchorage-dependent cells without interfering with gentle circulation of liquid growth medium in the bioreactor.

FIG. I

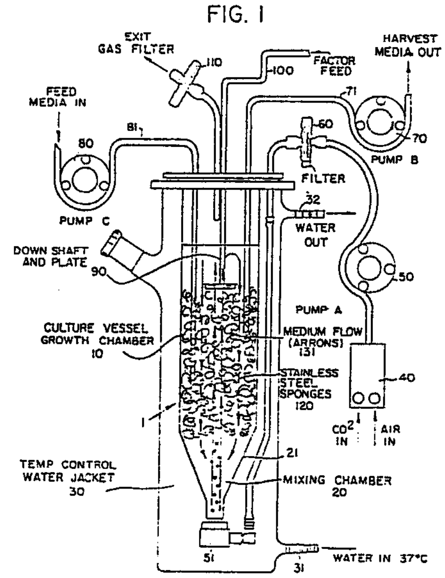


EP 0 276 154 A2

## Description

### Method and Apparatus for Cell Culture

FIELD OF THE INVENTION

This invention relates to airlift bioreactors which include a solid support material to facilitate cell culture as well as methods of operating same.

BACKGROUND OF THE INVENTION

Various types of airlift bioreactors are known. See for example, "Airlift Fermenters: Construction Behavior and Uses". Advances in Biotechnology Processes I. pages 67-95, 1983, Alan R. Liss. New York, New York.

A particular commercially-available airlift bioreactor which can be readily modified according to the present invention has an upper growth chamber which is connected to a lower smaller diameter mixing chamber via a downwardly and inwardly sloping conical section. A gas mixture is sparged into the mixing chamber and sets up a gentle circulation of liquid growth medium within the growth chamber.

Certain cell lines are anchorage dependent as is well known in the art. This means that the cells must be growth in an environment wherein they can be attached to a solid support material. Heretofore, this has been accomplished, for example, by attaching the cells to stacked petrie dishes or by growing the cells in roller bottles. Each of these approaches has drawbacks such as the inability to achieve high volumetric cell density or inadequate circulation of nutrients, etc. necessary to growth of the cells.

Another known method of growing anchorage dependent cells uses gel bead immobilization. Using this technique, the cells are immobilized within small beads formed from alginate gel, for example. The cell containing beads may be suspended in a liquid growth medium and just like suspension type cells may be circulated to provide adequate contact with nutrients and dissolved gases to promote cell growth.

SUMMARY OF THE INVENTION

According to the invention, airlift bioreactors are improved by including a packing material in their growth chambers for increasing cell density, providing a solid support for anchorage dependent cells and increasing gas dissolution into liquid growth medium circulating within the growth chamber.

Additionally, the invention provides methods of growing cells in airlift bioreactors having growth chambers which include a packing material. These methods relate to the growing of suspension cells as well as anchorage dependent cells.

BRIEF DESCRIPTION OF THE DRAWINGS

FIG. 1 shows an airlift bioreactor having a stainless steel solid support material in its growth chamber according to a preferred embodiment of the invention; and

FIG 2 is a graph showing daily production of receptor protein using the bioreactor shown in FIG. 1.

DESCRIPTION OF THE PREFERRED EMBODIMENT

As used herein the term "anchorage dependent cells" shall refer to any cells particularly mammalian, which will grow and multiply when attached to a solid support material and shall include but are not limited to cells which will only grow when attached to a solid support material. Examples of anchorage dependent cells which may be grown using the apparatus and methods according to the invention include but are not limited to Chinese Hamster Ovary cells, ATTC No. CCL-61.

The term "liquid growth medium" shall refer to any liquid medium in which cells may be grown. A particularly preferred growth medium for use in growing mammalian cells according to the present invention is Iscove's modified Dulbecco's medium supplemented with heat inactivated fetal calf serum.

The term "packing material" shall refer to any material placed within an airlift bioreactor which can serve as a solid support for the attachment of anchorage dependent cells and which will catch rising gas bubbles and suspended cells without totally disrupting gentle circulation of liquid growth medium within the bioreactor. Preferably, the packing material is finely divided into long thin strands, beads, etc. which imports a high surface area for a given volume. This finely divided state allows liquid growth medium to flow through the packing material and contact all the cells attached to it or trapped within it.

In this sense, cells grown in the bioreactor are essentially held stationary by the packing material while the liquid growth medium is in constant circulation.

Packing materials having a broad surface area such as extended baffles are unsuitable. Such large disruptions cause turbulence which necessitates higher driving forces to maintain circulation and also promote excessive foaming.

The ability of the packing material to catch gas bubbles is important because it increases the contact time between the gas and the liquid growth medium which improves gas dissolution and thereby allows the bioreactor to support an increased cell density.

Suitable packing materials include but are not limited to long filament like materials such as stainless steel wool, fiber glass (glass wool), and polymer strands as well as small beads of glass or plastic such as polyethylene, collagen beads, polysaccharide beads such as alginate or hydroxymethy cellulose beads etc. as well as mixtures of any of these. A particularly preferred packing material is stainless steel wool.

A preferred airlift bioreactor systems for use in the present invention is shown in Fig 1.. The system includes a bioreactor 1 within a temperature control water jacket 30. The temperature control water jacket 30 has a water inlet 31 for receiving temperature controlled water from suitable temperature control means (not shown). the temperature controlled water exits the temperature control jacket 30 via outlet 32. The temperature control jacket preferably maintains the bioreactor 1 at a preselected temperature suitable for cell growth.

The bioreactor 1 has from top to bottom a growth chamber 10 including packing means 120. The growth chamber 10 is connected via a downwardly and inwardly sloping conical section 21 to a mixing chamber 20 of small diameter than the growth chamber 10.

The preferred airlift bioreactor for use in the present invention is described in U.S. Patent Application Serial No. 711,932, filed March 15, 1985 the specification of which is incorporated herein by reference.

In operation the bioreactor 1 is filled with a liquid growth medium 130 suitable for culture of the particular cells to be grown.

First, cells to be grown in the bioreactor are introduced into the liquid growth medium 130. Then the liquid growth medium 130 is introduced into the bioreactor 1 by medium pump 80 via medium feed line 81 until the growth chamber 10 is partly filled.

Gas is then sparged up through the liquid growth medium 130 within the bioreactor 1 via gas sparger 51. The upwardly flowing gas constitutes a steam of gas bubbles which instigates a gentle circulation of liquid growth medium 130 within the bioreactor 1. This gentle circulation is denoted by arrows 131.

As can be seen, packing means 120 is positioned within bioreactor 1 so as to intersect at least a portion of the liquid growth medium 130 which is gently circulating within the bioreactor 1. Preferably, the packing means is positioned within the growth chamber 10 with no packing means being located in the conical section 21 or mixing chamber 20. Also, the liquid growth medium 130 is preferably filled to a level extending above the top of the packing means 120. To facilitate this, the packing means only extends part of the height of the growth chamber 10 and is held down by hold down means 90. Preferably, hold down means 90 is a shaft extending out through the top of the bioreactor having a lower end terminating in a plate atop the packing means 120 as shown. This hold down means in its preferred form is a hollow tube which provides a conduit into the bioreactor to which an external line may be connected, such as factor feed line 100 as shown.

The gentle circulation of liquid growth medium and cells is continued to ensure homogeneity within the bioreactor. Then, if desired, the gentle circulation can be briefly discontinued (by stopping the sparging), for a period sufficient to allow anchorage dependent cells to adhere to the packing means 120.

In operation, factors such as glucose, various nutrients, and sodium hydroxide to control the pH, etc. may be intrdouced as needed into growth chamber 10 via the factor feed line 100.

From time to time or on a continuous basis, spent liquid growth medium may be withdrawn from the bioreactor 1 via media harvest line 71 using pump 70 and replaced with fresh liquid growth medium via media feed line 81 and pump 80.

In the above manner, cells can be grown in the bioreactor, which cells are transfected for example, with a particular human gene for producing a particular biological product (e.g., protein, enzyme, hormone, chimeric antibody, etc.)which the cells will release into liquid growth medium 130. In accordance with one aspect of the invention, this biological product is then recovered from the harvested liquid growth medium using known methods.

Preferably, the gas being introduced into the bioreactor 1 is a mixture of $CO_2$ and oxygen-containing gas, such as air. Flow meter 40 is used to adjust in known manner the relative mixture of $co_2$ to air in known manner based on the oxygen demand of the cells being grown as well as the pH of the liquid growth medium 130 within bioreactor 1,while the total flow rate of both gases is controlled by pump 50.

The bioreactor may contain various sensors (not shown) for measuring the pH, temperature and oxygen content of the liquid growth medium 130. Additionally, these sensors may be connected to a preprogramable microprocessor (also not shown) for controlling in known manner the various pumps which have been illustrated herein.

The invention will now be described wih reference to the following example which is illustrative only and is not meant to limit the scope of the invention in any way. The example was performed as written.

## EXAMPLE

Chinese Hamster Ovary (CHO) cell line, obtained from the ATTC No. CCL-61 which were transfected with the human gene for the interlukin 2 (Il 2) receptor protein,were grown in an airlift bioreactor according to the following procedure.

An airlift bioreactor obtained from Bellco Glass Inc., Vineland, New Jersey was packed with six stainless steel sponges. The sponges were packed in such a way as to make a tight fit with the glass wall of the reactor. However, no sponges or parts thereof were in the mixing chamber of the reactor. The sponges were held securely in place with an adjustable shaft attached to the top of the reactor. The basic features of this bioreactor including the stainless steel sponges are as illustrated in Fig. 1.

Each of the stainless steel sponges was a "Kurly Kate" sanitary stainless steel sponge, #756 which was obtained from Purex Industrial, Lawrence, MA. Each sponge was made up of numerous strands of stainless steel in a coiled spring-like configuration. The strands were virtually continuous in length and measured approximately 0.016 inches in width and approximately 0.004 inches in thickness. The total surface area of a single sponge was calculated to be 3.396 square centimeters (cm2). Each sponge was rinsed in distilled water prior to being packed in the reactor. The total surface area of all six sponges in the reactor was calculated to be 20,376 cm2.

The reactor containing the sponges was then steam sterilized in an autoclave.

The liquid growth medium which was used was an Iscove's modified Dulbecco's medium, obtain from Gibco Laboratories #430-2200. It was supplemented with 2% heat-inactivated fetal calf serum which was obtained from Kansas City Biological.

The CHO cells were first grown in conventional roller bottles. Then some of these cells were suspended in the liquid growth medium using known procedures. This suspension of cells was then introduced into the sterile bioreactor. The liquid growth medium in the reactor was sparged at a rate of 50 cc-min for 5 minutes to obtain a homogeneous mixture of cells. The sparge was stopped for 45 minutes to facilitate attachment of the CHO cells to the stainless steel sponge. The sparge was then restarted.

The bioreactor was allowed to function continuously and held at a constant temperature of 37°C. The pH of the liquid growth medium, glucose consumption, lactate production and product (receptor protein) were monitored daily.

Periodically, spent medium containing receptor protein was removed from the bioreactor and replaced with fresh medium.

It was observed that cells attached readily to the stainless steel sponges. All cells appeared to become attached within the first half hour of incubation. The sponges acted as a substrate for the cells and also as a trap for air bubbles in the gas stream. This trapping action helped to increase issolved oxygen in the culture medium.

A gentle circulation pattern of liquid growth medium occurred as shown in Fig. 1, notwithstanding the positioning of the stainless steel sponges within the growth chamber. Thus the fluid dynamics of the bioreactor containing the stainless steel sponges was such as to create a homogeneous environment for the cells with respect to nutrients and dissolved oxygen.

The production rate of human receptor is shown in Fig. 2. Spent medium was collected and assayed in known manner by an ELISIA type assay for the presence of receptor protein. The bars in Fig. 2 represent the total amount of receptor protein harvested each day for the first 74 days of cell growth.

Production of Il2 receptor ranged from 12.5 to 320 milligrams per day which an average daily production of approximately 120 milligrams. In contrast, daily production of receptor protein by CHO cells grown in a conventional 850 cm2 roller bottle was found to be approximately 1.65 milligrams per day.

As can be seen from the forgoing example, the bioreactor apparatus incorporating the packing material according to the present invention is well suited to the growth of anchorage dependent cells such as the CHO cell line illustrated.

However, the apparatus of the present invention would also be suitable for the growth of suspension cells. Suspension cells would be trapped within the packing material much the same way air bubbles were trapped in the Example. In this way, the cell density of the suspension type cells within the bioreactor could be increased. Because the packing material does not impair the gentle circulation of liquid growth medium within the growth chamber, all cells which are located within the packing material are readily contacted with the circulating liquid growth medium containing nutrients and dissolved gases.

As mentioned previously, another advantage of the use of packing material in an airlift bioreactor according to the invention is the entrapment of air bubbles within the packing material. This entrapment provides longer contact time between the air bubbles and the liquid growth medium gently circulating within the bioreactor and thereby permits more gas to be dissolved within the liquid growth medium at a given sparging rate. That is, a greater amount of gas may be dissolved within the liquid growth medium for supporting greater numbers of cells within the bioreactor, without increasing the sparging rate beyond that which would provide a gentle circulation of liquid growth medium within the bioreactor. As it known in the art, sparging rates must be limited so as to avoid excessive amounts of forming which might disrupt the operation of the bioreactor.

The foregoing description of the preferred embodiment has been provided for the purpose of illustrating the invention but is not meant to limit the scope thereof to the particular embodiment described.

## Claims

1. An airlift bioreactor for growing cells which release biological products in a liquid growth medium, said bioreactor comprising:

a. a growth chamber for receiving the cells and the liquid growth medium and providing an environment for cell growth, said growth chamber having internal side walls which define a middle;

b means for gently bubbling a stream of gas up through the middle of said growth chamber to thereby cause gentle circulation of the liquid growth medium up through the middle of said growth chamber and then back down along the internal side walls of said growth chamber; and

c. packing means for increasing the density of cells within the bioreactor and facilitating absorption of at least a portion of the gas stream into the liquid growth medium, said packing means being located within said growth chamber in a position which intersects at least a portion of the gentle circulation of liquid growth medium present in said bioreactor when said bioreactor is in operation.

2. The air lift bioreactor of claim 1, wherein the internal side walls in said growth chamber are configured to define an inwardly sloping conical section at the bottom of the growth chamber for directing liquid growth medium which is flowing down the internal side walls back towards the middle of said growth chamber where it can again be uplifted.

3. The air lift bioreactor of claim 1, wherein the packing means comprises a stainless steel sponge material.

4. The air lift bioreactor of claim 2, wherein the packing means comprises a stainless steel sponge material.

5. The air lift bioreactor of claim 2, further comprising a mixing chamber located beneath said chamber which is continuous with the middle of said growth chamber, said mixing chamber including means for receiving the stream of gas to be bubbled up through the middle of said growth chamber.

6. The air lift bioreactor of claim 3, further comprising temperature control means for maintaining the liquid growth medium and the cells at a predetermined temperature within the growth chamber.

7. The air lift bioreactor of claim 6, further comprising gas stream supply means for supplying the gas stream to said bioreactor at a predetermined gentle flow rate.

8. The air lift bioreactor of claim 7, further comprising:

a) liquid growth medium feed means for feeding liquid growth medium into said growth chamber at predetermined intervals and in predetermined quantities; and

b) liquid growth medium removal means for removing liquid growth medium from said growth chamber at predetermined intervals and in predetermined quantities: whereby said air lift bioreactor may be continuously operated and biologocial products which are produced by the cells growing in said growth chamber can be harvested from liquid growth medium removed from said growth chamber.

9. The air lift bioreactor of claim 8, further comprising microprocessing control means for operating said temperature means, gas streams supply means, liquid growth medium feed means and liquid growth medium removal means according to predetermined operating instructions.

10. The air lift bioreactor of claim 8, wherein said packing means comprises stainless steel wool sponge material.

11. A method of growing anchorage dependent mammalian cells in an air lift bioreactor according to claim 1, said method comprising the steps of:

a) introducing liquid growth medium containing the anchorage dependent mammalian cells to be grown into the growth chamber of the bioreactor;

b) sparging a gas up through the middle of the growth chamber for a time sufficient to distribute the cells within the packing material within the growth chamber;

c) discontinuing the sparging for a time which is sufficient to allow the cells to attach themselves to the packing material in the bioreactor; and

d) sparging the liquid growth medium at a rate which is sufficient to provide gentle circulation of liquid growth medium within the growth chamber.

12. A bioreactor/fermentor which maintains cells or immobilized cells in suspension and whose geometry provides a mixing action to said cells with gentle aeration with a minimum of shear force, of the type comprising a reactor vessel having a mixing chamber, with a lower end and an open upper end, and a growth chamber, with an open lower end of said mixing chamber and the open lower end of said growth chamber coinciding with one another:

said mixing chamber having a first cylindrical side wall which defines the open upper end, a centrally located gas inlet means located in the lower end of said mixing chamber, and a bottom of the reactor vessel, for receiving the gas inlet means and, together with the first cylindrical side wall, for defining the lower end of said mixing chamber; and

said growth chamber comprising a conical side wall defining the open lower end of said growth chamber, the conical side wall sloping inwardly toward the open upper end of said mixing chamber, a second cylindrical side wall of larger diameter than the first cylindrical side wall of said mixing chamber, the second cylindrical side wall being located above the conical side wall, and top lid means for providing an air-tight seal on said growth chamber, the top lid means including gas vent means,

whereby oxygen-containing gas may be introduced into said reactor vessel through the centrally located gas inlet means and bubbled up through the cells and liquid medium to gently carry the cells and liquid medium upward from said mixing chamber to said growth chamber, flow downward along the conical side wall sloping inwardly towards the open upper end of said mixing chamber to replace the cells and liquid medium being carried upwards from said mixing chamber by the oxygen-containing gas, thereby providing a mixing action to said cells with aeration and a minimum of shear force while providing dissolved oxygen in the medium to support cell growth;

the improvement comprising a packing means for increasing cell density by trapping cells and for providing a site for the attachment of anchorage-dependent cells, said packing means being located in the growth chamber of said bioreactor.

13. The bioreactor of claim 12 wherein said reactor vessel is supported within a temperature control jacket means having fluid inlet and fluid outlet means.

14. A method for carrying out cell culture or fermentation in a reactor vessel having a cylindrical mixing chamber with an open upper end and a cylindrical growth chamber of larger diameter than the mixing chamber, the growth chamber having a packing means for trapping cells and a downward sloping conical sidewall defining an open lower end coinciding with the open upper end of the mixing chamber, wherein the cells are trapped by or attached to the packing means and the growth medium is gently circulated between the mixing chamber and growth chamber without any need for internal baffles and the liquid growth medium is aerated to provide sufficient oxygen to support cell growth while subjecting the cells to a minimum amount of shear force, said method comprising the steps of:

a) introducing a liquid growth medium into the reactor vessel to fill the mixing chamber and the growth chamber of the reactor vessel;

b) introducing a cell or immobilized cell population into the growth medium in the reactor vessel;

c) introducing a gentle stream of oxygen-containing gas into the center of the mixing chamber and bubbling the oxygen-containing gas upwardly from the mixing chamber into the growth chamber at a flow rate between about 10 to 300 cc/minute which is sufficient to lift cells and liquid growth medium from the mixing chamber up into the growth chamber, trapping the cells in the packing means and aerating the liquid growth medium thereby providing sufficient oxygen to support cell growth;

d) recirculating upwardly-lifted liquid growth medium from the growth chamber back to the mixing chamber while passing the liquid growth medium across the downward sloping conical sidewall of the growth chamber to sweep cells and liquid growth medium off of the conical sidewall of the growth chamber; and

e) growing the cells while maintaining the liquid growth medium and cells at a preselected incubation temperature.

15. The method of claim 14 wherein said cells are eukaryotic or prokaryotic.

16. The method of claim 14 wherein said incubation temperature is about 37° C.

17. The method of claim 14 wherein said cells are suspension cells.

18. The method of claim 17 wherein said cells are anchorage-dependent cells.

19. The method of claim 18 wherein said packing means comprise stainless steel wool.

20. The method of claim 14 wherein said gas stream comprises about 5% $CO_2$ and 95% air.

13 05 88

0276154

FIG. 1

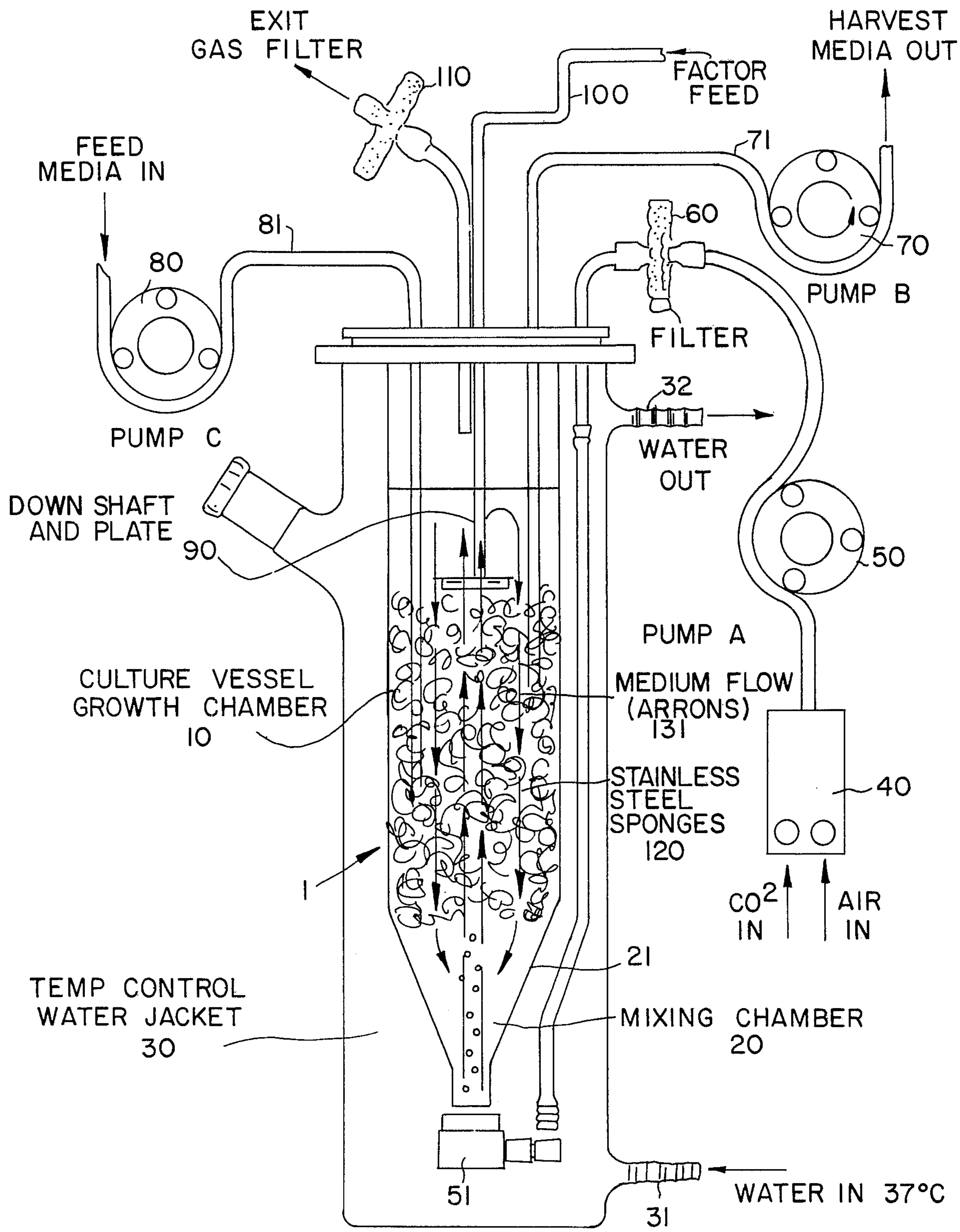

FIG. 2

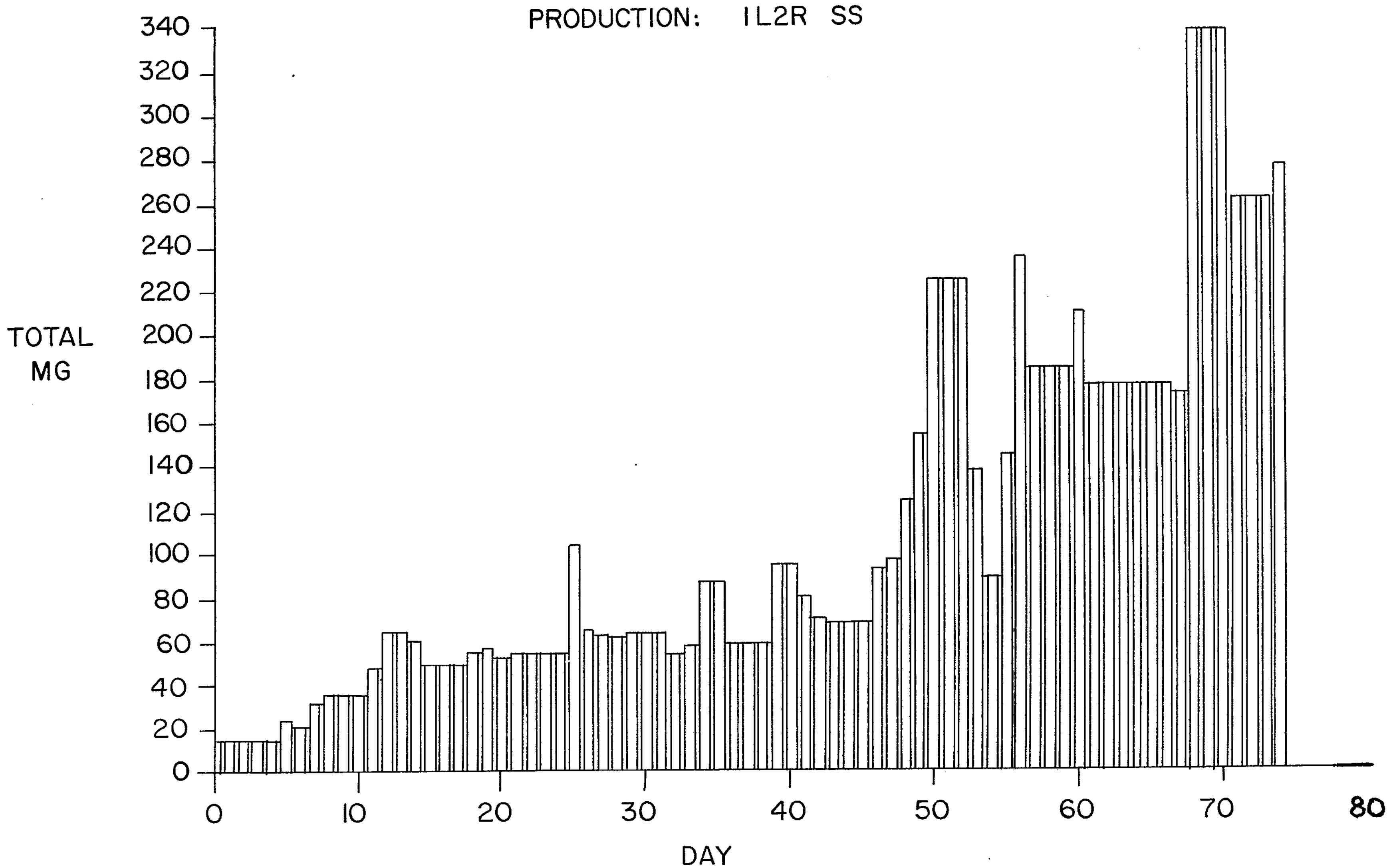
PRODUCTION: IL2R SS
TOTAL MG
340
320
300
280
260
240
220
200
180
160
140
120
100
80
60
40
20
0
0
10
20
30
40
50
60
70
80
DAY